(19) **Europäisches Patentamt**
**European Patent Office**
**Office européen des brevets**

(11) **EP 1 985 604 A1**

(12) **EUROPEAN PATENT APPLICATION**

(43) Date of publication:
**29.10.2008 Bulletin 2008/44**

(51) Int Cl.:
*C07C 67/36* *(2006.01)*      *C07C 69/14* *(2006.01)*
*C07C 51/12* *(2006.01)*      *C07C 53/08* *(2006.01)*

(21) Application number: **07251756.8**

(22) Date of filing: **26.04.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **BP Chemicals Limited
Sunbury-on-Thames, Middlesex TW16 7BP (GB)**

(72) Inventor: **The designation of the inventor has not
yet been filed**

(74) Representative: **Brooke, Caron
BP International Limited,
Patents & Agreements,
Chertsey Road
Sunbury-on-Thames,
Middlesex TW16 7LN (GB)**

(54) **Process for the production of methyl acetate**

(57)    Production of methyl acetate by carbonylating, under substantially anhydrous conditions, a dimethyl carbonate feed with carbon monoxide in the presence of a solid acid carbonylation catalyst and hydrogen.

EP 1 985 604 A1

**Description**

This invention relates to a process for preparing methyl acetate by reacting dimethyl carbonate with carbon monoxide in the presence of a solid acid carbonylation catalyst.

Liquid phase carbonylation processes, such as the carbonylation of methanol with carbon monoxide using homogeneous catalysts based on rhodium and/or iridium to produce acetic acid are well known and are operated on a commercial scale. Gas phase carbonylation processes for the carbonylation of feedstocks such as methanol and dimethyl ether using heterogeneous catalysts are also known.

EP-A-0 596 632 describes a vapour phase process for the carbonylation of methanol to produce acetic acid in the presence of a modified mordenite catalyst at high temperatures and pressures.

WO 01/07393 describes a process for the catalytic conversion of a feedstock comprising carbon monoxide and hydrogen to produce at least one of an alcohol, ether and mixtures thereof and reacting carbon monoxide with the at least one of an alcohol, ether and mixtures thereof in the presence of a catalyst selected from solid super acids, heteropolyacids, clays, zeolites and molecular sieves, in the absence of a halide promoter, under conditions of temperature and pressure sufficient to produce at least one of an ester, acid, acid anhydride and mixtures thereof. However, the use of zeolites to catalyse the carbonylation reaction is not exemplified.

WO 2005/105720 describes a process for production of a carboxylic acid and/or an ester or anhydride thereof by carbonylating an aliphatic alcohol or reactive derivative thereof with carbon monoxide in the substantial absence of halogens in the presence of a modified mordenite catalyst at a temperature in the range 250 to 600 °C and a pressure in the range 10 to 200 bar.

WO 2006/121778 describes a process for the production of a lower alkyl ester of a lower aliphatic carboxylic acid by carbonylating, under substantially anhydrous conditions, a lower alkyl ether, such as dimethyl ether with carbon monoxide in the presence of a mordenite or ferrierite catalyst.

Thermochimica Acta, 434 (2005), 88-92 discloses the thermal decomposition of dimethyl carbonate over solid acids and bases including H-ZSM-5 and non-zeolitic materials at a range of temperatures to produce dimethyl ether and smaller quantities of other decomposition products such as methanol.

In view of the above-mentioned prior art there remains the need for a heterogeneous carbonylation process for the production of methyl acetate using a solid acid catalyst which is superior to the afore-mentioned processes.

It has now been found that this can be achieved if the solid acid-catalysed heterogeneous carbonylation process is carried out in the presence of hydrogen and using dimethyl carbonate as the carbonylatable reactant.

Accordingly, the present invention provides a process for the production of methyl acetate which process comprises carbonylating, under substantially anhydrous conditions, a dimethyl carbonate feed with carbon monoxide in the presence hydrogen and a solid acid carbonylation catalyst.

The process of the present invention uses dimethyl carbonate as the carbonylatable feed. Dimethyl carbonate has a boiling point of 90 °C and a flash point of 16°C and is therefore liquid at ambient temperatures. Consequently, it is less hazardous to handle and transport, than, for example, dimethyl ether which has a boiling point of -25 °C and a flash point of -41 °C and which therefore needs to be handled either as a vapour or as a presurised liquid. The dimethyl carbonate may be employed in the carbonylation reaction either as a liquid or as a vapour.

The carbon monoxide may be substantially pure carbon monoxide, for example, carbon monoxide typically provided by suppliers of industrial gases, or it may contain impurities that do not interfere with the conversion of the dimethyl carbonate to methyl acetate, such as nitrogen, helium, argon, methane and/or carbon dioxide.

The process of the present invention is carried out in the presence of hydrogen. The hydrogen may be fed to the carbonylation process as a separate stream or as a mixture with carbon monoxide. Mixtures of hydrogen and carbon monoxide are commercially produced by the steam reforming of hydrocarbons and by the partial oxidation of hydrocarbons. Such mixtures are commonly referred to as synthesis gas. Synthesis gas comprises mainly carbon monoxide and hydrogen but may also contain smaller quantities of carbon dioxide.

Suitably, the molar ratio of carbon monoxide : hydrogen may be in the range 1 : 3 to 15 : 1, such as 1 : 1 to 10 : 1, for example, 1 : 1 to 4 : 1.

Suitably, hydrogen is present in the process at a partial pressure of at least 0.1 barg, such as in the range 1 to 50 barg.

Suitably, carbon monoxide is present in the process at a partial pressure of at least 0.1 barg, such as in the range 1 to 50 barg.

The molar ratio of dimethyl carbonate to carbon monoxide is suitably in the range 1 : 1 to 1 : 99, such as 1 : 1 to 1 : 30

The catalyst used in the process of the invention may be any solid acid which is effective to catalyse the carbonylation of dimethyl carbonate with carbon monoxide to produce methyl acetate. Suitable solid acid catalysts for use in the process of the present invention include zeolites.

Zeolites are available from commercial sources, generally in the Na form, $NH_4$ form or H-form of the zeolite.

The Na and NH$_4$ forms can be converted to the acid (H-form) by known techniques, such as calcination at high temperature. Alternatively, zeolites may be synthesised using known techniques.

Zeolites comprise a system of channels which may be interconnected with other channel systems or cavities such as side-pockets or cages. The ring structures are generally 12-member rings, 10-member rings or 8 member rings. A zeolite may possess rings of different sizes. The zeolites for use in the present invention preferably contain at least one channel which is defined by an 8-member ring. Most preferably, the 8-member ring channel is interconnected with at least one channel defined by a ring with 10 and/or 12 members. The window size of the channel systems should be such that the reactant dimethyl ether and carbon monoxide molecules can diffuse freely in and out of the zeolite framework. Suitably, the window size of an 8-member ring channel may be at least 2.5 x 3.6 Angstroms. The Atlas of Zeolite Framework Types (C. Baerlocher, W. M. Meier, D. H. Olson, 5th ed. Elsevier, Amsterdam, 2001) in conjunction with the web-based version (http://www.iza-structure.org/databases/) is a compendium of topological and structural details about zeolite frameworks, including the types of ring structures present in a zeolite and the dimensions of the channels defined by each ring type. Examples of zeolites suitable for use in the present invention include zeolites of framework type MOR, for example mordenite, FER, such as ferrierite, OFF, for example, offretite and GME, for example gmelinite.

For the process of the present invention it is preferred that the zeolite has a silica to alumina ratio of at least 5 but preferably less than or equal to 100, such as in the range 7 to 40, for example 10 to 30. Where the aluminium atoms have been replaced by framework modifier elements such as gallium, it is preferred that the ratio of silica : X$_2$O$_3$ where X is a trivalent element, such as aluminium, gallium, boron and/or iron is at least 5 and preferably less than or equal to 100, such as in the range 7 to 40, for example 10 to 30.

In one embodiment of the present invention the zeolite catalyst is a mordenite zeolite. The mordenite may be employed in the acid form (H-mordenite) or it may be optionally ion-exchanged or otherwise loaded with one or more metals such as copper, silver, nickel, iridium, rhodium, platinum, palladium or cobalt.

The metal loading on the mordenite zeolite may be expressed in terms of the fractional loading of the metal as gram atoms of metal per gram atom of aluminium in the mordenite. The metal loading can also be expressed as a mole percentage loading relative to aluminium in the mordenite through the relationship :

$$\text{mol\% Metal} = (\text{gram atoms Metal/gram atoms aluminium}) \times 100$$

Thus, for example, a loading of 0.55 gram atoms of copper per aluminium in the mordenite equates to a 55 mol% loading of copper relative to aluminium in the mordenite.

Suitably, the metal loading may be in the range of 1 to 200 mol% relative to aluminium, for example, 50 to 120 mol%, such as 50 to 110 mol%.

Suitably, the mordenite may be loaded with at least one of silver and copper and optionally additionally loaded with platinum. Where platinum is used in addition to silver and/or copper, it is preferably present in an amount in the range 0.05 to 5 mol% relative to aluminium.

The mordenite framework, may in addition to the silicon and aluminium atoms, contain additional trivalent elements, such as boron, gallium and/or iron.

Where the mordenite contains at least one or more trivalent framework, the metal loading in the mordenite can be expressed in terms of the fractional loading of the metal as gram atoms of metal per gram atom of total trivalent elements in the mordenite. The metal loading can also be expressed as a mole percentage loading relative to total trivalent elements in the mordenite through the relationship :

$$\text{mol\% Metal} = (\text{gram atoms Metal/gram atoms aluminium}) \times 100$$

It is preferred that the zeolite catalyst is activated immediately before use by heating the zeolite at elevated temperature for at least one hour under flowing nitrogen, carbon monoxide or hydrogen.

Because the carbonylation reaction is to be conducted substantially in the absence of water, it is preferred that the zeolite catalyst is dried prior to use. The catalyst may be dried, for example by heating to a temperature of 400 to 500 °C.

The process is carried out under substantially anhydrous conditions, i.e in the substantial absence of water. Thus, in the process of the present invention, water is kept as low as is feasible. To accomplish this, the dimethyl carbonate and carbon monoxide reactants (and catalyst) are preferably dried prior to introduction into the process. However, small amounts of water may be tolerated without adversely affecting the formation of methyl acetate. Suitably, water may be present in the dimethyl carbonate feed in an amount of 2.5 wt% or less, such as 0.5 wt% or less

The process of the present invention is suitably carried out at a temperature in the range of 150 °C to 500 °C, for example, in the range 150 to 350 °C.

The process of the present invention may be carried out at a total reaction pressure of 1 to 100 barg, for example, 10 to 100 barg, such as 20 to 80 barg.

The Gas Hourly Space Velocity (GHSV) is suitably in the range 500 to 15,000 h$^{-1}$, such as 2000 to 10,000 h$^{-1}$.

The process of the present invention is suitably carried out by passing dimethyl carbonate vapour, carbon monoxide and hydrogen through a fixed, fluidised or moving bed of the solid acid catalyst maintained at the desired temperature and pressure.

Preferably, the process of the present invention is carried out substantially in the absence of halides, such as iodide. By the term 'substantially' is meant that the halide, for example, iodide content of the reactant gases and catalyst is less than 500 ppm, preferably less than 100 ppm.

The primary product of the process is methyl acetate but small amounts of acetic acid may also be produced. The methyl acetate produced by the process of the present invention can be removed in the form of a vapour and thereafter condensed to a liquid.

The methyl acetate may be recovered and sold as such or it may be forwarded to other chemical processes. Where the methyl acetate is recovered from the carbonylation reaction products, some or all of it may be hydrolysed to form acetic acid. Alternatively, the entire carbonylation reaction product may be passed to a hydrolysis stage and acetic acid separated thereafter. The hydrolysis may be carried out by known techniques such as reactive distillation in the presence of an acid catalyst.

The process may be operated as either a continuous or a batch process, preferably as a continuous process.

The invention is now illustrated with reference to the following Examples.

## Catalyst Preparation

### Preparation of Cu(55)Pt(1)-Mordenite - Catalyst I

H-mordenite (120 g) with a silica to alumina ratio of 20 (ex Süd-chemie) was weighed into a 500 mL round bottomed flask together with 21.7 g of copper (II) nitrate hemipentahydrate (98% ACS) and a stirrer bar. Sufficient deionised water (ca. 150 mL) was then added to the flask until a thick slurry was obtained. The top of the flask was then covered loosely and the flask left to stir overnight. The zeolite was then dried under reduced vacuum using a rotary evaporator before being dried in an oven at 90 °C for 12 hours. To a portion of this copper loaded mordenite (10 g) in a round bottomed flask was added a solution of $K_2Pt(NO_3)_2$ (0.065g, ex Aldrich) in water (30 mL). The top of the flask was then covered loosely and the flask left to stir overnight. The zeolite was then dried under reduced vacuum using a rotary evaporator before being dried in an oven at 90 °C for 12 hours. The zeolite was then calcined in a muffle oven (oven volume = 18L) under a static atmosphere of air at 500 °C for 24 h. The zeolite was then compacted at 12 tonnes in a 33 mm die set using a Specac Press, and crushed and sieved to a particle size fraction of 212 to 335 microns. This zeolite is referred to as Cu(55)Pt(1)-MOR as the Cu loading is ca. 55 mole % and the Pt loading is ca 1 mole % relative to Al contained in the mordenite.

### Preparation of Ag(55)-Mordenite - Catalyst II

H-mordenite (100 g) with a silica to alumina ratio of 19 (ex Tosoh) was weighed into a 500 mL round bottomed flask together with 13.8 g of silver nitrate (99+% ACS, ex Aldrich) and a stirrer bar. Sufficient deionised water (ca. 100 mL) was then added to the flask until a thick slurry was obtained. The top of the flask was then covered loosely and the flask left to stir overnight. The zeolite was then dried under reduced vacuum using a rotary evaporator before being dried in an oven at 110 °C for 18 hours. The zeolite was then calcined in a muffle oven (oven volume =18L) under a static atmosphere of air at 500 °C for 24 h. The zeolite was then compacted at 12 tonnes in a 33 mm die set using a Specac Press, and crushed and sieved to a particle size fraction of 250 to 500 microns. This zeolite is referred to as Ag(55)-MOR as the Ag loading is ca. 55 mole % relative to Al contained in the mordenite.

## Example 1

A Hastelloy reactor tube was packed with a Cu(55)Pt(1)MOR (catalyst I) (0.6 mL, particle size 250 to 500 microns) and a pre-bed of gamma alumina (0.2 g). The section of the reactor tube containing the catalyst was fitted with an electrical heating jacket. The reactor tube and heating jacket were mounted in a heated cabinet maintained at 130°C. Carbon monoxide, hydrogen and nitrogen gas feeds were provided to the reactor. The gases were fed via mass flow controllers to a gas header where they were mixed. The header pressure was regulated by a back pressure regulator. The gas mixture was fed to the reactor from the gas header via a mass flow controller. The reactor pressure was regulated by a back pressure regulator.

The catalyst was conditioned in three stages, first under nitrogen, then under carbon monoxide and finally under carbon monoxide and hydrogen. Nitrogen was was fed through the prebed and reactor at atmospheric pressure at a rate of 50 mL min-1 (NTP) for ca. 3 h during which time the reactor was heated to 300 °C at rate of 3 °C min$^{-1}$. The reactor pressure was raised to 19.4 barg and carbon monoxide was fed at a rate of 50 mL min-1 (NTP) for 2 h. A mixture of carbon monoxide, 38.0 mL min-1 (NTP), hydrogen, 9.9 mL min-1 (NTP), and nitrogen, 0.2 mL min-1 (NTP) was then fed to the reactor for 2h.

After the catalyst had been conditioned dimethyl carbonate was fed to the reactor by pumping liquid dimethyl carbonate via a mass flow controller through a heated mixing valve to generate dimethyl carbonate vapour at a rate of 2.5 mL min-1 (NTP) and was combined with the gas feed in the mixing valve before the combined feed was passed over the prebed and catalyst. The feed rates for all components and the partial pressures for components once the dimethyl carbonate feed has begun are shown in Table 1. The time zero for the reaction is taken as the time when dimethyl carbonate feed started.

A constant flow of reaction off-gases was taken from the high pressure side of the reactor system through a needle valve, let down to atmospheric pressure while maintaining a temperature of at least 130°C and passed to a gas chromatograph for analysis. The rate of production of methyl acetate and acetic acid was measured from the gas chromatography analysis of the vapour phase reactor effluent. The space time yield (STY) of acetyls products was calculated as the molar equivalent weight of acetic acid corresponding to the sum of the methyl acetate and acetic acid produced expressed as grams of acetic acid per hour per litre of catalyst and is shown in Table 1 after 10, 20 and 40 hours on stream.

## Examples 2-4 and Comparative Examples A and B

Example 1 was repeated except that after the catalyst conditioning steps different gas compositions and/or catalyst were used while dimethyl carbonate was fed as shown in Table 1. The space time yield of acetyls products from these examples is also shown in Table 1.

**Table 1**

| Example | 1 | 2 | A | 3 | 4 | B |
|---|---|---|---|---|---|---|
| Catalyst | I | I | I | II | II | II |
| $H_2$ gas flow NTP ml min$^{-1}$ | 9.9 | 23.8 | 0.0 | 9.9 | 23.8 | 9.9 |
| CO gas flow NTP ml min$^{-1}$ | 38.0 | 23.5 | 37.9 | 38.0 | 23.5 | 38.0 |
| $N_2$ gas flow NTP ml min$^{-1}$ | 0.2 | 0.6 | 9.8 | 0.2 | 0.6 | 0.2 |
| DMC gas flow NTP ml min$^{-1}$ | 2.5 | 2.5 | 2.5 | 2.5 | 2.5 | 2.5 |
| Inlet pressure barg | 19.4 | 19.4 | 19.4 | 19.4 | 19.4 | 19.4 |
| Partial pressure $H_2$ barg | 4.0 | 9.6 | 0.0 | 4.0 | 9.6 | 0.0 |
| Partial pressure CO barg | 15.4 | 9.5 | 15.3 | 15.4 | 9.5 | 15.4 |
| Partial pressure $N_2$ barg | 0.1 | 0.3 | 4.0 | 0.1 | 0.3 | 4.0 |
| Partial pressure DMC barg | 1.0 | 1.0 | 1.0 | 1.0 | 1.0 | 1.0 |
| Temperature °C | 300 | 300 | 300 | 300 | 300 | 300 |
| **STY (AcOH eq g l$^{-1}$ h$^{-1}$) 10 h** | **388** | **264** | **179** | **277** | **261** | **187** |
| **STY (AcOH eq g l$^{-1}$ h$^{-1}$) 20 h** | **351** | **252** | **116** | **234** | **222** | **102** |
| **STY (AcOH eq g l$^{-1}$ h$^{-1}$) 40 h** | **314** | **248** | **75** | **190** | **185** | **64** |
| **40 h STY as % of STY at 10 h** | **81** | **94** | **42** | **69** | **71** | **34** |

Comparing Example 1 with Comparative Example A, and Example 3 with Comparative Example B, it can be seen that in Examples 1 and 3 where hydrogen is present, the activity of the catalyst is substantially greater than in Comparative Examples A and B where no hydrogen is used.

Furthermore, the rate of decline of activity is less in Examples 1-4 than in the Comparative Examples A and B. For example after 40 hours on stream the activity in Comparative Example A has declined to 42% of its activity at 10 hours whilst the activity in Example 1 only declined to 81% of its activity at 10 hours.

**Claims**

1. A process for the production of methyl acetate which process comprises carbonylating, under substantially anhydrous conditions, a dimethyl carbonate feed with carbon monoxide in the presence of a solid acid carbonylation catalyst and hydrogen.

2. A process according to claim 1 wherein the carbon monoxide and hydrogen are present in a molar ratio in the range 1 : 3 to 15 : 1.

3. A process according to claim 2 wherein the carbon monoxide and hydrogen are present in a molar ratio in the range 1 : 1 to 10 : 1.

4. A process according to claim 3 wherein the carbon monoxide and hydrogen are present in a molar ratio in the range 1 : 1 to 4 : 1.

5. A process according to any one of the preceding claims wherein the hydrogen is present at a partial pressure of at least 0.1 barg.

6. A process according to claim 5 wherein the hydrogen is present at a partial pressure in the range 1 to 50 barg.

7. A process according to any one of the preceding claims wherein the carbon monoxide is present at a partial pressure of at least 0.1 barg.

8. A process according to claim 7 wherein the carbon monoxide is present at a partial pressure in the range 1 to 50 barg.

9. A process according to any one of the preceding claims wherein the dimethyl carbonate and carbon monoxide are present in a molar ratio in the range 1 : 1 to 99 : 1.

10. A process according to claim 9 wherein the dimethyl carbonate and carbon monoxide are present in a molar ratio in the range 1 : 1 to 30 : 1.

11. A process according to any one of the preceding claims wherein the solid acid catalyst is a zeolite.

12. A process according to claim 11 wherein the zeolite contains at least one channel which is defined by an 8-member ring.

13. A process according to claim 12 wherein the 8-member ring channel is interconnected with at least one channel defined by a ring with 10 and/or 12 members.

14. A process according to claim 12 or claim 13 wherein the 8-member ring channel has a window size of at least 2.5 x 3.6 Angstroms.

15. A process according to any one of claims 11 to 14 wherein the zeolite has a silica : $X_2O_3$ ratio in the range 5 to 100, X being selected from at least one of aluminium, gallium, boron and iron.

16. A process according to any one of claims 11 to 15 wherein the zeolite has a framework type selected from the group consisting of MOR, FER, OFF and GME.

17. A process according to claim 16 wherein the zeolite is selected from the group consisting of mordenite, ferrierite, offretite and gmelinite.

18. A process according to claim 17 wherein the mordenite is H-mordenite.

19. A process according to claim 17 wherein the mordenite is ion-exchanged or otherwise loaded with at least one metal selected from the group consisting of copper, nickel, iridium, silver, rhodium, platinum, palladium and cobalt.

20. A process according to claim 19 wherein the mordenite is ion-exchanged or otherwise loaded with a metal selected from copper, silver and mixtures thereof.

21. A process according to claim 20 wherein the mordenite is additionally ion-exchanged or otherwise loaded with platinum.

22. A process according to claim 19 or claim 20 wherein a metal is present in the mordenite at a loading in the range of 1-200 mol% relative to aluminium.

23. A process according to claim 22 wherein the metal loading is 50 to 120 mol% relative to aluminium.

24. A process according to claim 21 wherein the platinum is present in an amount in the range 0.05 to 5 mol% relative to aluminium.

25. A process according to any one of claims 18 to 24 wherein the framework of the mordenite comprises gallium, iron or mixtures thereof.

26. A process according to any one of the preceding claims wherein the process is carried out in the substantial absence of halides.

27. A process according to any one of the preceding claims wherein the carbon monoxide and hydrogen is produced by the steam reforming of a hydrocarbon or the partial oxidation of a hydrocarbon.

28. A process according to any one of the preceding claims wherein the process is carried out at a temperature in the range 150 to 500 °C

29. A process according to claim 28 wherein the process is carried out at a temperature in the range 150 to 350 °C.

30. A process according to any one of the preceding claims wherein the process is carried out at a pressure in the range 1 to 100 barg.

31. A process according to claim 30 wherein the pressure is in the range 20 to 80 barg.

32. A process according to any one of the preceding claims wherein at least some of the methyl acetate product is hydrolysed to acetic acid.

33. A process according to claim 1 wherein the process is carried out at a temperature in the range of 150 °C to 350 °C in the presence of a mordenite catalyst and wherein carbon monoxide and hydrogen are present in a molar ratio in the range 1 : 1 to 10 : 1.

34. A process according to claim 1 wherein the process is carried out at a temperature in the range of 150 °C to 350 °C in the presence of a mordenite catalyst and wherein the hydrogen is present at a partial pressure in the range 1 to 50 barg.

EP 1 985 604 A1

**European Patent Office**

**EUROPEAN SEARCH REPORT**

Application Number

EP 07 25 1756

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 2006/121778 A (UNIV CALIFORNIA [US]; BP CHEM INT LTD [GB]; CHEUNG PATRICIA [US]; IGLE) 16 November 2006 (2006-11-16)<br>* paragraphs [0001], [0027] *<br>* page 11 - page 13 * | 1 | INV.<br>C07C67/36<br>C07C69/14<br>C07C51/12<br>C07C53/08 |
| A | WO 99/54273 A (BP CHEM INT LTD [GB]; LAW DAVID JOHN [GB]; LUCY ANDREW RICHARD [GB]; S) 28 October 1999 (1999-10-28)<br>* page 3, line 30 - page 5, line 7 *<br>* page 6, line 1 - line 3; compound (D) * | 1 | |
| L | US 7 253 304 B1 (TUSTIN GERALD CHARLES [US]) 7 August 2007 (2007-08-07)<br>* the whole document * | | |

TECHNICAL FIELDS SEARCHED (IPC)

C07C

The present search report has been drawn up for all claims

| Place of search | Date of completion of the search | Examiner |
|---|---|---|
| The Hague | 27 September 2007 | Kardinal, Siegmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

8

# EP 1 985 604 A1

## ANNEX TO THE EUROPEAN SEARCH REPORT
## ON EUROPEAN PATENT APPLICATION NO.

EP 07 25 1756

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2007

| Patent document cited in search report | | Publication date | Patent family member(s) | | Publication date |
|---|---|---|---|---|---|
| WO 2006121778 | A | 16-11-2006 | US 2006252959 A1 | | 09-11-2006 |
| | | | US 2006287551 A1 | | 21-12-2006 |
| WO 9954273 | A | 28-10-1999 | AU 3436299 A | | 08-11-1999 |
| | | | EP 1071647 A1 | | 31-01-2001 |
| | | | TW 565551 B | | 11-12-2003 |
| US 7253304 | B1 | 07-08-2007 | NONE | | |

For more details about this annex : see Official Journal of the European Patent Office, No. 12/82

EPO FORM P0459

9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0596632 A
- WO 0107393 A
- WO 2005105720 A
- WO 2006121778 A

### Non-patent literature cited in the description

- *Thermochimica Acta,* 2005, vol. 434, 88-92
- **C. BAERLOCHER ; W. M. MEIER ; D. H. OLSON.** The Atlas of Zeolite Framework Types. Elsevier, 2001